# EUROPÄISCHE PATENTSCHRIFT

(11) **EP 1 830 855 B1**
(45) Veröffentlichungstag und Bekanntmachung des Hinweises auf die Patenterteilung: **24.02.2010**
(21) Anmeldenummer: 05821797.7
(22) Anmeldetag: 13.12.2005
(51) Int. Cl.: A61K 31/5377, A61K 9/20, A61K 9/28

(54) **FESTE, ORAL APPLIZIERBARE PHARMAZEUTISCHE DARREICHUNGSFORMEN ENTHALTEND RIVAROXABAN MIT MODIFIZIERTER FREISETZUNG**
SOLID, ORALLY APPLICABLE PHARMACEUTICAL ADMINISTRATION FORMS CONTAINING RIVAROXABAN HAVING MODIFIED RELEASE
PRODUITS PHARMACEUTIQUES SOLIDES, ADMINISTRES PAR VOIE ORALE ET CONTENANT DU RIVAROXABAN, A LIBERATION MODIFIEE

(30) Priorität: 24.12.2004 DE 102004062475
(43) Veröffentlichungstag der Anmeldung: 12.09.2007
(73) Patentinhaber: Bayer Schering Pharma Aktiengesellschaft, 13353 Berlin (DE)
(72) Erfinder: BENKE, Klaus, 51465 Bergisch Gladbach (DE); HENCK, Jan-Olav, 47877 Willich (DE)
(86) Internationale Anmeldenummer: PCT/EP2005/013337
(87) Internationale Veröffentlichungsnummer: WO 2006/072367

(56) Entgegenhaltungen:
- WO-A-03/035133
- US-A1- 2003 153 610
- WEINZ CORINNA ET AL: "Metabolism and distribution of (14C)BAY 59-7939 - An oral, direct factor Xa inhibitor - In rat, dog and human" DRUG METABOLISM REVIEWS, Bd. 36, Nr. Suppl. 1, August 2004 (2004-08), Seite 98, XP009063377 & 7TH INTERNATIONAL MEETING OF THE INTERNATIONAL SOCIETY FOR THE STUDY OF XENOBIOTICS; VANCOUVER, BC, CANADA; AUGUST 29-SEPTEMBER 02, 2004 ISSN: 0360-2532
- KUBITZA ET AL: "Safety, pharmacodynamics, and pharmacokinetics of single doses of BAY 59-7939, an oral, direct factor Xa inhibitor" CLINICAL PHARMACOLOGY & THERAPEUTICS, MOSBY-YEAR BOOK, ST LOUIS, MO, US, Bd. 78, Nr. 4, Oktober 2005 (2005-10), Seiten 412-421, XP005127187 ISSN: 0009-9236

## Beschreibung

Die vorliegende Erfindung betrifft feste, oral applizierbare, 5-Chlor-*N*-({(5*S*)-2-oxo-3-[4-(3-oxo-4-morpholinyl)-phenyl]-1,3-oxazolidin-5-yl}-methyl)-2-thiophencarboxamid enthaltende pharmazeutische Darreichungsformen mit modifizierter Freisetzung sowie Verfahren zu ihrer Herstellung, ihre Anwendung als Arzneimittel, ihre Verwendung zur Prophylaxe, Sekundärprophylaxe und/oder Behandlung von Erkrankungen sowie ihre Verwendung zur Herstellung eines Arzneimittels zur Prophylaxe, Sekundärprophylaxe und/oder Behandlung von Erkrankungen.

Unter modifiziert freisetzenden Darreichungsformen werden erfindungsgemäß solche Zubereitungen verstanden, deren Wirkstofffreisetzungscharakteristik nach der Einnahme bezüglich Zeit, Verlauf und/oder Ort im Magen-Darm-Trakt so eingestellt ist, wie sie nach Applikation konventioneller Formulierungen (z.B. orale Lösungen oder den Wirkstoff schnell freisetzende feste Darreichungsformen) nicht erreicht werden kann. Neben dem Begriff "modifizierte Freisetzung" werden häufig auch alternative Begriffe wie "retardierte", "verzögerte" oder "kontrollierte Freisetzung" verwendet. Diese sind vom Umfang der vorliegenden Erfmdung ebenfalls mit umfasst.

Für die Herstellung modifiziert freisetzender pharmazeutischer Darreichungsformen sind verschiedene Methoden bekannt, siehe beispielsweise B. Lippold in "Oral Controlled Release Products: Therapeutic and Biopharmaceutic Assessment" Hrsg. U. Gundert-Remy und H. Möller, Stuttgart, Wiss.Verl.-Ges., 1989, 39-57.

5-Chlor-*N*-({(5*S*)-2-oxo-3-[4-(3-oxo-4-morpholinyl)-phenyl]-1,3-oxazolidin-5-yl}-metyl)-2-thiophencarboxamid (I) ist ein niedermolekularer, oral applizierbarer Inhibitor des Blutgerinnungsfaktors Xa, der zur Prophylaxe, Sekundärprophylaxe und/oder Behandlung verschiedener thromboembolischer Erkrankungen eingesetzt werden kann (siehe hierzu WO-A 01/47919). Wenn im Folgenden vom Wirkstoff (I) die Rede ist, so sind dabei alle Kristallmodifikationen und die amorphe Form von 5-Chlor-*N*-({(5*S*)-2-oxo-3-[4-(3-oxo-4-morpholinyl)-phenyl]-1,3-oxazolidin-5-yl}-methyl)-2-thiophencarboxamid (I) sowie die jeweiligen Hydrate, Solvate und Co-Kristallisate mit umfasst.

Bei Krankheiten, die über einen längeren Zeitraum behandelt werden müssen, oder zur längerfristigen Prophylaxe von Krankheiten ist es wünschenswert, die Häufigkeit der Einnahme von Medikamenten so gering wie möglich zu halten. Dies ist nicht nur bequemer für den Patienten, sondern es erhöht auch die Behandlungssicherheit (compliance), indem es die Nachteile unregelmäßiger Einnahmen vermindert. Die gewünschte Reduktion der Einnahmefrequenz, beispielsweise von zweimal täglicher auf einmal tägliche Applikation, kann über eine Verlängerung der therapeutisch effektiven Plasmaspiegel durch modifizierte Wirkstofffreigabe aus den Darreichungsformen erreicht werden.

Nach Einnahme von Darreichungsformen mit modifizierter Wirkstofffreisetzung kann außerdem durch Glättung des Plasmaspiegelverlaufes (Minimierung des sogenannten peak-trough-Verhältnisses), also durch Vermeidung von hohen Plasmawirkstoffkonzentrationen, die häufig nach Gabe schnellfreisetzender Arzneiformen zu beobachten sind, das Auftreten unerwünschter, mit den Konzentrationsspitzen korrelierten Nebenwirkungen vermindert werden.

Insbesondere für die Dauertherapie oder -prophylaxe und Sekundärprophylaxe arterieller und/oder venöser thromboembolischer Erkrankungen (beispielsweise tiefe Venenthrombosen, Schlaganfall, Myokardinfarkt und Lungenembolie) ist es von Vorteil, den Wirkstoff (I) in einer Form zur Verfügung zu haben, die über eine modifizierte Wirkstofffreigabe zu einer Verringerung des peaktrough-Verhältnisses führt und eine einmal tägliche Applikation ermöglicht.

Bei der Formulierungsentwicklung sind weiterhin die physikalisch-chemischen und biologischen Eigenschaften des Wirkstoffes (I) zu berücksichtigen, beispielsweise die relativ geringe Wasserlöslichkeit (ca. 7 mg/L; 25°C), der relativ hohe Schmelzpunkt von ca. 230°C des Wirkstoffes (I) in der Kristallmodifikation, in der der Wirkstoff (I) bei der Herstellung nach dem in WO 01/47919 unter Beispiel 44 beschriebenen Weg erhalten wird und die im Folgenden als Modifikation I bezeichnet wird, und die Plasmahalbwertszeit von ca. 7 Stunden. Für die gewünschte einmal tägliche Applikatiopn sind demnach spezielle galenische Formulierungen notwendig, die den Wirkstoff (I) unter Berücksichtigung seiner physikochemischen und biologischen Eigenschaften modifiziert freisetzen.

In DE 10355461 sind pharmazeutische Darreichungsformen beschrieben, die den Wirkstoff (I) in hydrophylisierter Form enthalten. Bevorzugt sind dabei schnell freisetzende Tabletten, die gemäß USP-Freisetzungsmethode mit Apparatur 2 (Paddle) einen Q-Wert (30 Minuten) von 75 % besitzen.

Überraschenderweise wurde nun gefunden, dass Darreichungsformen, die den Wirkstoff (I) mit bestimmter, definierter modifizierter Rate freisetzen, eine einmal tägliche Applikation bei vergleichsweise konstanten Plasmakonzentrationen ermöglichen.

Gegenstand der vorliegenden Erfindung sind feste, oral applizierbare pharmazeutische Darreichungsformen mit modifizierter Freisetzung, enthaltend 5-Chlor-*N*-({(5*S*)-2-oxo-3-[4-(3-oxo-4-morpholinyl)-phenyl]-1,3-oxazolidin-5-yl}-methyl)-2-thiophencarboxamid (I), dadurch gekennzeichnet, dass 80 % des Wirkstoffes (I) (bezogen auf die deklarierte Gesamtmenge des Wirkstoffes) über einem Zeitraum von mindestens 2 und höchstens 24 Stunden gemäß USP-Freisetzungsmethode mit Apparatur 2 (Paddle) freigesetzt werden.

In einer bevorzugten Ausführungsform der vorliegenden Erfindung werden 80 % des Wirkstoffes (I) in einem Zeitraum von 4 bis 20 Stunden gemäß USP-Freisetzungsmethode mit Apparatur 2 (Paddle) freigesetzt.

Der Wirkstoff (1) kann in den erfindungsgemäßen pharmazeutischen Darreichungsformen in kristalliner Form oder in nicht-kristalliner amorpher Form vorliegen oder in Mischungen von kristallinen und amorphen Wirkstoffanteilen.

Enthalten die erfindungsgemäßen Darreichungsformen den Wirkstoff (I) in kristalliner Form, wird in einer bevorzugten Ausführungsform der vorliegenden Erfindung der Wirkstoff (I) in mikronisierter Form der Kristallmodifikation I eingesetzt. Hierbei besitzt der Wirkstoff (I) vorzugsweise eine mittlere Partikelgröße X₅₀ kleiner 10 µm, insbesondere kleiner 8 µm; sowie einen X₉₀-Wert (90 %-Anteil) kleiner 20 µm, insbesondere kleiner 15 µm.

In einer weiteren bevorzugten Ausführungsform der vorliegenden Erfindung liegt im Fall der Verwendung von kristallinem Wirkstoff (I) der mikronisierte Wirkstoff (I) in hydrophylisierter Form vor, wodurch seine Lösegeschwindigkeit erhöht ist. Die Herstellung von hydrophylisiertem Wirkstoff (I) ist in DE 10355461, ausführlich dargestellt.

Vorzugsweise liegt der Wirkstoff (I) in den erfindungsgemäßen pharmazeutischen Darreichungsformen aber nicht in kristalliner Form sondern vollständig oder mit überwiegendem Anteil in amorpher Form vor. Ein großer Vorteil der Amorphisierung des Wirkstoffes (I) ist die Erhöhung der Wirkstofflöslichkeit und damit die Möglichkeit, die Absorptionsquote des Wirkstoffes (I), insbesondere aus tieferen Darmabschnitten zu erhöhen.

Zur Amorphisierung des Wirkstoffes (I) sind verschiedene pharmazeutisch geeignete Herstellmethoden denkbar.

Hierbei ist die Lösemethode, bei der ein Wirkstoff und gegebenenfalls eingesetzte Hilfsstoff(e) gelöst und dann weiterverarbeitet werden, weniger gut geeignet, da der kristalline Wirkstoff (I) nur eine begrenzte Löslichkeit in pharmazeutisch geeigneten organischen Lösemitteln wie beispielsweise Aceton oder Ethanol aufweist und deshalb unverhältnismäßig große Lösemittelmengen verwendet werden müssen.

Die erfindungsgemäß bevorzugte Methode zur Amorphisierung des Wirkstoffes (I) ist das Schmelzverfahren, bei der ein Wirkstoff zusammen mit oder in einem oder mehreren geeigneten Hilfsstoffen geschmolzen wird.

Besonders bevorzugt ist dabei das Schmelzextrusionsverfahren [ Breitenbach, J., "Melt extrusion: from process to drug delivery technology", European Journal of Pharmaceutics and Biopharmaceutics 54 (2002), 107-117; Breitenbach, J., "Feste Lösungen durch Schmelzextrusion - ein integriertes Herstellkonzept", Pharmazie in unserer Zeit 29 (2000), 46-49 ].

Durch Wahl einer geeigneten Rezeptur und geeigneter Herstellparameter ist bei diesem Verfahren sicherzustellen, dass der Wirkstoffabbau pharmazeutisch akzeptable Grenzen nicht überschreitet. Dies ist bei einem Schmelzpunkt von ca. 230°C des Wirkstoffes (I) in der Kristallmodifikation I eine schwierige Aufgabe, da in diesem hohen Temperaturbereich in der Regel signifikante Zersetzungsraten des Wirkstoffes und/oder der Hilfsstoffe zu erwarten sind.

Das Schmelzextrusionsverfahren zur Herstellung des Wirkstoffes (I) in amorpher Form wird erfindungsgemäß in Gegenwart eines Polymers wie beispielsweise Polyvinylpyrrolidone, Polyethylenglycole (PEG), Polymethacrylate, Polymethylmethacrylate, Polyethylenoxide (insbesondere wasserlösliche Polyethylenoxid-Harze wie z. B. POLYOX™ Water Soluble Resins, Dow), Polyoxyethylen-Polyoxypropylen-Blockcopolymere, Vinylpyrrolidon-Vinylacetat-Copolymerisate oder eines Celluloseethers wie beispielsweise Hydroxypropylcellulose (HPC) oder eines Gemisches verschiedener Polymere wie beispielsweise Gemische von zwei oder mehr der genannten Polymere durchgeführt. Bevorzugtes Polymer ist dabei Hydroxypropylcellulose (HPC), Polyvinylpyrrolidon (PVP) oder ein Gemisch von HPC und PVP. Besonders bevorzugt ist das Polymer dabei Hydroxypropylcellulose (HPC) oder Polyvinylpyrrolidon (PVP).

Der Polymer-Anteil im Schmelzextrudat beträgt erfindungsgemäß vorzugsweise mindestens 50 % der Gesamtmasse des Schmelzextrudates.

Der Wirkstoff (I) liegt im Schmelzextrudat erfindungsgemäß vorzugsweise in einer Konzentration zwischen 1 und 20 %, bezogen auf die Gesamtmasse des Schmelzextrudates, vor.

Beim Schmelzextrusionsverfahren zur Herstellung des Wirkstoffes (I) in amorpher Form hat es sich als vorteilhaft erwiesen, einen oder mehrere pharmazeutisch geeignete Stoffe zur Erniedrigung der Schmelztemperatur des Wirkstoffes (I) bzw. als Weichmacher zuzusetzen, um den während des Extrusionsprozesses erfolgenden Wirkstoffabbau zu verringern und die Verarbeitung zu erleichtern.

Vorzugsweise werden diese pharmazeutisch geeigneten Stoffe erfindungsgemäß in einer Konzentration von 2 bis 40 %, bezogen auf die Gesamtmasse des Schmelzextrudates zugesetzt.

Dafür geeignet sind beispielsweise Harnstoff, Polymere wie Polyvinylpyrrolidone, Polyethylenglycole, Polymethacrylate, Polymethylmethacrylate, Polyoxyethylen-Polyoxypropylen-Blockcopolymere, Vinylpyrrolidon-Vinylacetat-Copolymerisate oder Zuckeralkohole wie beispielsweise Erythritol, Maltitol, Mannitol, Sorbitol und Xylitol. Bevorzugt werden Zuckeralkohole eingesetzt. Durch Wahl geeigneter Herstellparameter ist dabei sicherzustellen, dass der Wirkstoff (I) möglichst vollständig in den amorphen Zustand überführt wird, um die Wirkstofflöslichkeit zu erhöhen.

Das durch Schmelzextrusionsverfahren gewonnene, den Wirkstoff (I) enthaltende Extrudat wird geschnitten, gegebenenfalls ausgerundet und/oder lackiert und kann beispielsweise zu einer Sachetformulierung weiterverarbeitet oder in Kapseln abgefüllt werden (multiple-unit Formulierungen). Eine weitere Möglichkeit besteht darin, das nach Schmelzextrusion erhaltene Extrudat nach dem Schneiden und Mahlen mit üblichen Tablettierhilfsstoffen zu mischen, zu Tabletten zu verpressen und diese gegebenenfalls anschließend noch zu lackieren (single-unit Formulierungen).

Verschiedene den Wirkstoff (I) modifiziert freisetzende, pharmazeutische orale Darreichungsformen können erfindungsgemäß eingesetzt werden. Ohne den Umfang der vorliegenden Erfindung zu beschränken, seien dafür beispielhaft und vorzugsweise genannt:
1. Tablettenformulierungen ("single-units") basierend auf Erosionsmatrix-Systemen
2. Multipartikuläre Darreichungsformen mit Erosions- und/oder diffusionskontrollierter Freisetzungskinetik wie Granulate, Pellets, Mini-Tabletten und daraus hergestellte Arzneiformen wie beispielsweise Sachets, Kapseln oder Tabletten
3. Darreichungsformen basierend auf osmotischen Freisetzungssystemen

### 1. Tablettenformulierungen basierend auf Erosionsmatrix-Systemen

Die modifizierte Wirkstoffreisetzung erfolgt hierbei durch Formulierung des Wirkstoffes in einer erodierbaren Matrix aus einem oder mehreren löslichen Polymeren, wobei die Wirkstofffreisetzung abhängig ist von der Quell- und Auflöse- bzw. Erosionsrate der Matrix sowie der Lösegeschwindigkeit, Löslichkeit und Diffusionsrate des Wirkstoffes. Dieses Prinzip zur modifizierten Wirkstofffreigabe ist auch unter den Begriffen Erosionsmatrix- oder Hydrokolloidmatrix-System bekannt. Das Erosions-/Hydrokolloid-Matrix-Prinzip zur Modifizierung der Wirkstofffreisetzung pharmazeutischer Darreichungsformen ist beispielsweise beschrieben in:
- Alderman, D.A., "A review of cellulose ethers in hydrophilic matrixes for oral controlled-release dosage forms", Int. J. Pharm. Tech. Prod. Mfr. 5 (1984), 1-9.
- Melia, C.D., "Hydrophilic matrix sustained release systems based on polysaccharide carriers", Critical Reviews in Therapeutic Drug Carrier Systems 8 (1991), 395-421.
- Vazques, M.J. et al, "Influence of technological variables on release of drugs from hydrophilic matrices", Drug Dev. Ind. Pharm. 18 (1992), 1355-1375

Die gewünschte Freisetzungskinetik kann beispielsweise über den Polymertyp, die Polymerviskosität, die Polymer- und/oder Wirkstoffpartikelgröße, das Wirkstoff-Polymer-Verhältnis sowie Zusätze weiterer pharmazeutisch üblicher Hilfsstoffe wie beispielsweise lösliche oder/und unlösliche Füllstoffe gesteuert werden.

Als Matrixbildner eignen sich im Rahmen der vorliegenden Erfindung zahlreiche Polymere, beispielsweise Polysaccharide und Celluloseether wie Methylcellulose, Carboxymethylcellulose, Hydroxyethylmethylcellulose, Ethylhydroxyethylcellulose, Hydroxyethylcellulose, wobei bevorzugt Hydroxypropylcellulose (HPC) oder Hydroxypropylmethylcellulose (HPMC) oder Gemische von Hydroxypropylcellulose und Hydroxypropylmethylcellulose eingesetzt werden.

Der Matrixbildner ist in den erfindungsgemäßen Tablettenformulierungen basierend auf Erosionsmatrix-Systemen vorzugsweise in einer Konzentration zwischen 10 und 95 %, bezogen auf die Gesamtmasse der Tablette enthalten.

Der Wirkstoff (I) ist in den erfindungsgemäßen Tablettenformulierungen basierend auf Erosionsmatrix-Systemen vorzugsweise in einer Konzentration zwischen 1 und 50 %, bezogen auf die Gesamtmasse der Tablette enthalten.

Neben dem/den Polymer(en) zur Bildung der Erosions-(Hydrokolloid)matrix und dem Wirkstoff können den Tablettenformulierungen weitere dem Fachmann geläufige Tablettier-Hilfsstoffe zugesetzt werden (z.B. Bindemittel, Füllstoffe, Schmier-/Gleit-/Fließmittel). Die Tabletten können zudem mit einem Lack überzogen werden.

Geeignete Materialien für einen Lichtschutz- und/oder Farblack sind beispielsweise Polymere wie Polyvinylalkohol, Hydroxypropylcellulose und/oder Hydroxypropylmethylcellulose, gegebenenfalls in Kombination mit geeigneten Weichmachern wie beispielsweise Polyethylenglycol oder Polypropylenglycol und Pigmenten wie beispielsweise Titandioxid oder Eisenoxide.

Weiterhin als Materialien für die Herstellung eines Lackes geeignet sind beispielsweise wässrige Dispersionen, wie z. B. Ethylcellulose-Dispersion (z.B. Aquacoat, FMC) oder eine Poly(ethylacrylat, methylmethacrylat)-Dispersion (Eudragit NE 30 D, Röhm/Degussa). Außerdem können dem Lack Weichmacher und Netzmittel zugesetzt werden (z.B. Trietylcitrat oder Polysorbate), Anti-Klebemittel wie beispielsweise Talkum oder Magnesiumstearat und hydrophile Porenbildner wie beispielsweise Hydroxypropylmethyl-cellulose, Polyvinylpyrrolidon oder Zucker. Der Lack bewirkt im wessentlicher, dass es nach Applikation während der ersten ein bis maximal zwei Stunden zu einer Verzögerung der Wirkstofffreisetzung kommen kann.

Weiterhin als Materialien für die Herstellung eines Lackes geeignet sind Stoffe zur Erzielung einer Magensaftresistenz wie beispielsweise anionische Polymere auf Basis von Methacrylsäure (Eudragit L+S, Röhm/Degussa) oder Celluloseacetatphthalat.

Zur Herstellung von erfindungsgemäßen Tablettenformulierungen, enthaltend den Wirkstoff (I) in kristalliner oder überwiegend kristalliner Form eignen sich die üblichen, dem Fachmann bekannten Methoden wie Direkttablettierung, Tablettierung nach Trockengranulation, Schmelzgranulation, Extrusion oder Feuchtgranulation wie beispielsweise Wirbelschichtgranulation.

Vorzugsweise wird für die erfindungsgemäßen Tablettenformulierungen basierend auf Erosionsmatrix-Systemen der Wirkstoff (I) aber in amorpher oder überwiegend amorpher Form, insbesondere als Schmelzextrudat, eingesetzt, so dass der Wirkstoff (I) in der fertigen Formulierung in amorpher Form vorliegt.

Weiterer Gegenstand der vorliegenden Erfindung ist ein Verfahren zur Herstellung der erfindungsgemäßen Tablettenformulierung basierend auf Erosionsmatrix-Systemen, wobei vorzugsweise mit Hilfe der Schmelzextrusion ein den Wirkstoff (I) enthaltendes Extrudat hergestellt wird, welches dann gemahlen, mit weiteren dem Fachmann bekannten Tablettierhilfsstoffen (Matrixformer, Bindemittel, Füllstoffe, Schmier-/Gleit-/Fließmittel) gemischt und anschließend vorzugsweise mittels Direkttablettierung zu Tabletten verpresst wird, die abschließend mit einem Lack überzogen werden können.

### 2. Multipartikuläre Darreichungsformen wie Granulate, Pellets, Mini-Tabletten und daraus hergestellte Kapseln, Sachets und Tabletten

Neben den unter 1. beschriebenen so genannten "single-unit" (Einzelkörper)- Darreichungsformen sind für den Wirkstoff (I) auch multipartikuläre Darreichungsformen geeignet, deren modifizierte Wirkstofffreisetzung erosions-/diffusionskontrolliert erfolgt. Unter dem Begriff "multipartikuläre Darreichungsformen" werden erfindungsgemäß solche Formulierungen verstanden, die im Gegensatz zu "single-units" (Tabletten) aus mehreren kleinen Partikeln wie Granulatkörnern, sphärischen Granulaten (Pellets) oder Minitabletten bestehen. Der Durchmesser dieser Partikel beträgt in der Regel zwischen 0,5 und 3,0 mm, vorzugsweise zwischen 1,0 und 2,5 mm.

Der Vorteil dieser multipartikulären Systeme im Vergleich zu den single-units besteht in einer meist geringer ausgeprägten intra- und interindividuellen Variabilität der Magen-Darm-Passage mit daraus resultierender geringerer Variabilität der Plasmaprofile und oft auch reduzierter Nahrungsmittelabhängiskeit (food effect), d. h. verringerte Unterschiede nach Gabe auf gefüllten bzw. nüchternen Magen. Die Granulate (Pellets) oder kleinformatigen Tabletten (Mini-Tabletten mit max. 3 mm Durchmesser) können in Kapseln abgefüllt oder als Sachet zubereitet werden. Eine weitere Möglichkeit besteht in der Weiterverarbeitung zu größeren Tabletten, die nach Kontakt mit Wasser/Magensaft durch schnellen Zerfall die Primärgranulate/-pellets freigeben.

Für die Herstellung multipartikulärer pharmazeutischer Darreichungsformen, enthaltend den Wirkstoff (I), eignen sich grundsätzlich alle unter 1. genannten Hilfsstoffe und Verfahren.

Bevorzugt wird dabei als Matrixformer ein Polymer aus der Gruppe der Celluloseether eingesetzt, insbesondere Hydroxypropylcellulose (HPC) oder Hydroxypropylmethylcellulose (HPMC) oder ein Gemisch von Hydroxypropylcellulose und Hydroxypropylmethylcellulose.

Das Polymer ist in den erfindungsgemäßen pharmazeutischen Darreichungsformen basierend auf multipartikulären Darreichungsformen vorzugsweise in einer Konzentration zwischen 10 und 99 %, insbesondere zwischen 25 und 95 %, bezogen auf die Gesamtmasse der Zusammensetzung enthalten.

Der Wirkstoff (I) ist in den erfindungsgemäßen pharmazeutischen Darreichungsformen basierend auf multipartikulären Darreichungsformen vorzugsweise in einer Konzentration zwischen 1 und 30 %, bezogen auf die Gesamtmasse der Zusammensetzung enthalten.

Für die Herstellung von Pellets, die den Wirkstoff (I) in kristalliner oder überwiegend kristalliner Form enthalten, ist insbesondere das Extrusions-/Spheronisierungs-Verfahren geeignet, welches beispielsweise in Gandhi, R., Kaul, C.L., Panchagnula, R., "Extrusion and spheronization in the development of oral controlled-release dosage forms", Pharmaceutical Science & Technology Today Vol. 2, No. 4 (1999), 160-170 beschrieben ist.

In einer bevorzugten Ausführungsform der vorliegenden Erfindung enthalten die multipartikulären Darreichungsformen den Wirkstoff (I) in amorpher Form und werden dabei vorzugsweise mit Hilfe des Schmelzextrusionsverfahrens hergestellt.

Die Partikel/Pellets/Minitabletten können gegebenenfalls lackiert werden, beispielsweise mit wässrigen Dispersionen wie z. B. Ethylcellulose-Dispersion (z.B. Aquacoat, FMC) oder einer Poly(ethylacrylat, methylmethacrylat)-Dispersion (Eudragit NE 30 D, Röhm/Degussa). Außerdem können dem Lack Weichmacher und Netzmittel zugesetzt werden (z.B. Trietylcitrat oder Polysorbate), Anti-Klebemittel wie beispielsweise Talkum oder Magnesiumstearat und hydrophile Porenbildner wie beispielsweise Hydroxypropylmethylcellulose, Polyvinylpyrrolidon oder Zucker. Der Lack bewirkt in wesentlichen, dass es nach Applikation während der ersten ein bis maximal zwei Stunden zu einer Verzögerung der Wirkstofffreisetzung kommen kann.

Weiterhin als Materialien für die Herstellung eines Lackes geeignet sind Stoffe zur Erzielung einer Magensaftresistenz wie beispielsweise anionische Polymere auf Basis von Methacrylsäure (Eudragit L+S, Röhm/Degussa) oder Celluloseacetatphthalat.

Weiterer Gegenstand der vorliegenden Erfindung sind pharmazeutische Darreichungsformen, vorzugsweise Kapseln, Sachets oder Tabletten, enthaltend die oben beschriebenen multipartikulären Darreichungsformen.

Weiterer Gegenstand der vorliegenden Erfindung ist ein Verfahren zur Herstellung der erfindungsgemäßen multipartikulären pharmazeutischen Darreichungsformen, wobei vorzugsweise durch Schmelzextrusion ein den Wirkstoff (I) in amorpher Form enthaltendes Extrudat erhalten wird. In einer bevorzugten Ausführungsform der vorliegenden Erfindung wird durch Schneiden dieses Extrudatstranges und gegebenenfalls anschließendes Ausrunden direkt eine pelletförmige multipartikuläre Darreichungsform hergestellt. Die so erhaltenen Pellets können anschließend mit einem Lack überzogen werden und in Kapseln oder als Sachet abgefüllt werden.

### 3. Osmotische Freisetzungssysteme

Weitere geeignete Darreichungsformen, die den Wirkstoff (I) modifiziert freisetzen, basieren auf osmotischen Freisetzungssystemen. Hierbei werden Kerne, beispielsweise Kapseln oder Tabletten, vorzugsweise Tabletten, mit einer semipermeablen Membran umgeben, die mindestens eine Öffnung aufweist. Die wasserdurchlässige Membran ist für die Komponenten des Kerns undurchlässig, erlaubt aber den Eintritt von Wasser von außen über Osmose in das System. Das eingedrungene Wasser setzt dann über den entstehenden osmotischen Druck den Wirkstoff gelöst oder suspendiert aus der oder den Öffnung/en in der Membran frei. Die Gesamtwirkstofffreisetzung und die Freisetzungsrate können im Wesentlichen über die Dicke und Porosität der semipermeablen Membran, die Zusammensetzung des Kerns und die Anzahl und Größe der Öffnung/en gesteuert werden. Vorteile, Formulierungsaspekte, Anwendungsformen und Informationen zu Herstellverfahren sind u. a. in folgenden Publikationen beschrieben:
- Santus, G., Baker, R.W., "Osmotic drug delivery: a review of the patent literature", Journal of Controlled Release 35 (1995), 1-21
- Verma, R.K., Mishra, B., Garg, S., "Osmotically controlled oral drug delivery", Drug Development and Industrial Pharmacy 26 (7), 695-708 (2000)
- Verma, R.K., Krishna, D.M., Garg, S., "Formulation aspects in the development of osmotically controlled oral drug delivery systems", Journal of Controlled Release 79 (2002), 7-27
- US 4,327,725, US 4,765,989, US 20030161882, EP 1 024 793.

Für den Wirkstoff (I) sind sowohl Einkammersysteme (elementary osmotic pump) als auch Zweikammersysteme (push-pull-Systeme) geeignet. Der Wirkstoff (I) kann in den osmotischen Systemen sowohl in kristalliner, vorzugsweise mikronisierter Form als auch in amorpher Form vorliegen oder in Mischungen mit kristallinen und amorphen Anteilen.

Die Hülle des osmotischen Arzneimittelfreisetzungssystems besteht sowohl beim Einkammersystem als auch beim Zweikammersystem aus einem wasserdurchlässigen, für die Komponenten des Kerns undurchlässigen Material. Solche Hüllmaterialien sind im Prinzip bekannt und beispielsweise beschrieben in der EP-B 1-1 024 793, Seite 3-4. Erfindungsgemäß bevorzugt werden als Hüllmaterial Celluloseacetat oder Gemische von Celluloseacetat und Polyethylenglycol eingesetzt.

Auf die Hülle kann bei Bedarf ein Lack, beispielsweise ein Lichtschutz- und/oder Farblack aufgebracht werden. Geeignete Materialien hierfür sind beispielsweise Polymere wie Polyvinylalkohol, Hydroxypropylcellulose und/oder Hydroxypropylmethylcellulose, gegebenenfalls in Kombination mit geeigneten Weichmachern wie beispielsweise Polyethylenglycol oder Polypropylenglycol und Pigmenten wie beispielsweise Titandioxid oder Eisenoxide.

Beim osmotischen Einkammersystem enthält der Kern vorzugsweise:
- 2 bis 30 % Wirkstoff (I),
- 20 bis 50 % Xanthan,
- 10 bis 30 % eines Vinylpyrrolidon-Vinylacetat-Copolymers,
wobei gegebenenfalls die Differenz zu 100 % durch einen oder mehrere zusätzliche Bestandteile gebildet wird, die ausgewählt sind aus der Gruppe von weiteren hydrophilen, quellbaren Polymeren, osmotisch aktiven Zusätzen und pharmazeutisch üblichen Hilfsstoffen. Die Summe der Kernbestandteile beträgt 100 % und die %- Angaben beziehen sich jeweils auf die Gesamtmasse des Kerns.

Das osmotische Einkammersystem enthält als einen der wesentlichen Bestandteile des Kerns das hydrophile wasserquellbare Polymer Xanthan. Dabei handelt es sich um ein anionisches Heteropolysaccharid, das im Handel beispielsweise unter der Bezeichnung Rhodigel^{®} (hergestellt durch Rhodia) erhältlich ist. Es liegt in einer Menge von 20 bis 50 %, bevorzugt von 25 bis 40 %, bezogen auf die Gesamtmasse der Kernbestandteile vor.

Ein weiterer wesentlicher Bestandteil des Kerns ist das Vinylpyrrolidon-Vinylacetat-Copolymer. Dieses Copolymer ist an sich bekannt und kann mit beliebigen Mischungsverhältnissen der Monomere hergestellt werden. Das bevorzugt verwendete kommerziell erhältliche Kollidon^{®} VA64 (hergestellt durch BASF) ist z.B. ein 60:40- Copolymerisat. Es weist im allgemeinen einen Gewichtsmittelwert des Molekulargewichts Mw, bestimmt durch Lichtstreuungsmessungen, von etwa 45.000 bis etwa 70.000 auf. Die Menge des Vinylpyrrolidon-Vinylacetat-Copolymers im Kern beträgt 10 bis 30 %, bevorzugt 15 bis 25 %, bezogen auf die Gesamtmasse der Kernbestandteile.

Gegebenenfalls im Kern zusätzlich vorhandene hydrophile quellbare Polymere sind beispielsweise Hydroxypropylcellulose, Hydroxypropylmethylcellulose, Natriumcarboxymethylcellulose, Natriumcarboxymethylstärke, Polyacrylsäuren bzw. deren Salze.

Gegebenenfalls im Kern zusätzlich vorhandene osmotisch aktive Zusätze sind beispielsweise alle wasserlöslichen Stoffe, deren Verwendung in der Pharmazie unbedenklich ist, wie z.B. die in Pharmakopöen oder in "Hager" und "Remington Pharmaceutical Science" erwähnten wasserlöslichen Hilfsstoffe. Insbesondere können wasserlösliche Salze von anorganischen oder organischen Säuren oder nichtionische organische Stoffe mit großer Wasserlöslichkeit wie z.B. Kohlehydrate, insbesondere Zucker, Zuckeralkohole oder Aminosäuren verwendet werden. Zum Beispiel können die osmotisch aktiven Zusätze ausgewählt werden aus anorganischen Salzen wie Chloriden, Sulfaten, Carbonaten und Bicarbonaten von Alkali- oder Erdalkalimetallen, wie Lithium, Natrium, Kalium, Magnesium, Calcium sowie Phosphate, Hydrogen- oder Dihydrogenphosphate, Acetate, Succinate, Benzoate, Citrate oder Ascorbate davon. Des weiteren können Pentosen, wie Arabinose, Ribose oder Xylose, Hexosen, wie Glucose, Fructose, Galactose oder Mannose, Disaccharide wie Sucrose, Maltose oder Lactose oder Trisaccharide wie Raffinose verwendet werden. Zu den wasserlöslichen Aminosäuren zählen Glycin, Leucin; Alanin oder Methionin. Erfindungsgemäß besonders bevorzugt wird Natriumchlorid verwendet. Die osmotisch aktiven Zusätze sind bevorzugt in einer Menge von 10 bis 30 %, bezogen auf die Gesamtmasse der Kernbestandteile enthalten.

Gegebenenfalls im Kern zusätzlich vorhandene pharmazeutisch übliche Hilfsstoffe sind beispielsweise Pufferstoffe wie Natriumbicarbonat, Bindemittel wie Hydroxypropylcellulose, Hydroxypropylmethylcellulose und/oder Polyvinylpyrrolidon, Schmiermittel wie Magnesiumstearat, Netzmittel wie Natriumlaurylsulfat und/oder Fließregulierungsmittel wie hochdisperses Siliziumdioxid.

Weiterer Gegenstand der vorliegenden Erfindung ist ein Verfahren zur Herstellung eines erfindungsgemäßen osmotischen Einkammersystems, wobei die Komponenten des Kerns miteinander vermischt werden, gegebenenfalls feucht oder trocken granuliert werden, anschließend tablettiert werden und der so entstandene Kern mit der Hülle beschichtet wird, die gegebenenfalls noch mit einem Lichtschutz- und/oder Farblack überzogen wird und die mit einer oder mehreren Öffnungen versehen wird.

In einer bevorzugten Ausführungsform der vorliegenden Erfindung werden bei der Herstellung des osmotischen Einkammersystems die Kernkomponenten einer Feuchtgranulation unterzogen, da dieser Verfahrensschritt eine bessere Benetzbarkeit der Bestandteile des Tablettenkerns bewirkt, wodurch die eintretende Gastrointestinalflüssigkeit den Kern besser durchdringt, was vielfach zu einer rascheren und vollständigeren Freisetzung des Wirkstoffs führt.

Beim osmotischen Zweikammersystem besteht der Kern aus zwei Schichten, einer Wirkstoffschicht und einer Osmoseschicht. Ein derartiges osmotisches Zweikammersystem ist beispielsweise in DE 34 17 113 C2, ausführlich beschrieben.

Die Wirkstoffschicht enthält vorzugsweise:
- 1 bis 40 % Wirkstoff (I),
- 50 bis 95 % von einem oder mehreren osmotisch aktiven Polymeren, vorzugsweise Polyethylenoxid mittlerer Viskosität (40 bis 100 mPa · s; 5 %ige wässrige Lösung, 25°C; vorzugsweise gemessen mit einem geeigneten Brookfield Viskosimeter und einer geeigneten Spindel bei einer geeigneten Drehzahl, insbesondere mit einem Brookfield Viskosimeter Modell RVT und einer Spindel Nr. 1 bei einer Drehzahl von 50 U/min oder mit einem vergleichbaren Modell unter den entsprechenden Bedingungen (Spindel, Drehzahl)).

Die Osmoseschicht enthält vorzugsweise:
e 40 bis 90 % von einem oder mehreren osmotisch aktiven Polymeren, vorzugsweise Polyethylenoxid hoher Viskosität (5000 bis 8000 mPa · s; 1 %ige wässrige Lösung, 25°C; vorzugsweise gemessen mit einem geeigneten Brookfield Viskosimeter und einer geeigneten Spindel bei einer geeigneten Drehzahl insbesondere mit einem Brookfield Viskosimeter Modell RVF und einer Spindel Nr. 2 bei einer Drehzahl von 2 U/min oder mit einem vergleichbaren Modell unter den entsprechenden Bedingungen (Spindel, Drehzahl)).
• 10 bis 40 % eines osmotisch aktiven Zusatzes,
wobei die Differenz zu 100 % in den einzelnen Schichten unabhängig voneinander jeweils durch einen oder mehrere zusätzliche Bestandteile in Form von pharmazeutisch üblichen Hilfsstoffen gebildet wird. Die %-Angaben beziehen sich jeweils auf die Gesamtmasse der jeweiligen Kernschicht.

Im Kern des osmotischen Zweikammersystems können die gleichen osmotisch aktiven Zusätze wie im oben beschriebenen Fall des Einkammersystems verwendet werden. Bevorzugt ist hierbei Natriumchlorid.

Im Kern des osmotischen Zweikammersystems können die gleichen pharmazeutisch üblichen Hilfsstoffe wie im oben beschriebenen Fall des Einkammersystems verwendet werden. Bevorzugt sind hierbei Bindemittel wie Hydroxypropylcellulose, Hydroxypropylmethylcellulose und/oder Polyvinylpyrrolidon, Schmiermittel wie Magnesiumstearat, Netzmittel wie Natriumlaurylsulfat und/oder Fließregulierungsmittel wie hochdisperses Siliziumdioxid sowie ein Farbpigment wie Eisenoxid in einer der beiden Schichten zur Differenzierung von Wirkstoff- und Osmoseschicht.

Weiterer Gegenstand der vorliegenden Erfindung ist ein Verfahren zur Herstellung des erfindungsgemäßen osmotischen Zweikammersystems, wobei die Komponenten der Wirkstoffschicht gemischt und granuliert werden, die Komponenten der Osmoseschicht gemischt und granuliert werden und anschließend beide Granulate auf einer Zweischichttablettenpresse zu einer Zweischichttablette verpresst werden. Der so entstandene Kern wird dann mit einer Hülle beschichtet, die Hülle wird auf der Wirkstoffseite mit einer oder mehreren Öffnungen versehen und anschließend gegebenenfalls noch mit einem Lack überzogen.

In einer bevorzugten Ausführungsform der vorliegenden Erfindung werden bei der Herstellung des osmotischen Zweikammersystems sowohl die Komponenten der Wirkstoffschicht als auch die Komponenten der Osmoseschicht jeweils trocken granuliert, insbesondere mit Hilfe der Walzengranulation.

Erfindungsgemäß bevorzugt sind aufgrund der physikalisch-chemischen Eigenschaften des Wirkstoffes (I) osmotische Zweikammersysteme (push-pull-Systeme), bei denen die Wirkstoff- und Osmoseschicht getrennt vorliegen, beispielhaft und vorzugsweise als 2-Schichttablette formuliert. Die Vorteile gegenüber osmotischen Einkammersystemen sind hierbei die über einen längeren Zeitraum gleichmäßigere Freisetzungsrate sowie die Möglichkeit, den systembedingt notwendigen Wirkstoffüberschuss zu verringern.

Weiterer Gegenstand der vorliegenden Erfindung sind Arzneimittel, enthaltend eine erfindungsgemäße feste, oral applizierbare, den Wirkstoff (I) enthaltende pharmazeutische Darreichungsform mit modifizierter Freisetzung.

Weiterer Gegenstand der vorliegenden Erfindung ist die Verwendung der erfmdungsgemäßen festen, oral applizierbaren pharmazeutischen Darreichungsform mit modifizierter Freisetzung, enthaltend den Wirkstoff (I) zur Prophylaxe, Sekundärprophylaxe und/oder Behandlung von Erkrankungen, insbesondere von arteriellen und/oder venösen thromboembolischen Erkrankungen wie Myocardinfarkt, Angina Pectoris (eingeschlossen instabile Angina), Reokklusionen und Restenosen nach einer Angioplastie oder aortokoronarem Bypass, Schlaganfall, transitorische ischämische Attacken, periphere arterielle Verschlusskrankheiten, Lungenembolien oder tiefen Venenthrombosen.

Weiterer Gegenstand der vorliegenden Erfindung ist die Verwendung der erfindungsgemäßen festen, oral applizierbaren, den Wirkstoff (I) enthaltende pharmazeutischen Darreichungsform mit modifizierter Freisetzung, zur Herstellung eines Arzneimittels zur Prophylaxe, Sekundärprophylaxe und/oder Behandlung von Erkrankungen, insbesondere von arteriellen und/oder venösen thromboembolischen Erkrankungen wie Myocardinfarkt, Angina Pectoris (eingeschlossen instabile Angina), Reokklusionen und Restenosen nach einer Angioplastie oder aortokoronarem Bypass, Schlaganfall, transitorische ischämische Attacken, periphere arterielle Verschlusskrankheiten, Lungenembolien oder tiefen Venenthrombosen.

Weiterer Gegenstand der vorliegenden Erfindung ist die Verwendung von 5-Chlor-*N*-({(5*S*)-2-oxo-3-[4-(3-oxo-4-morpholinyl)-phenyl]-1,3-oxazolidin-5-yl}-methyl)-2-thiophencarboxamid (I) zur Herstellung einer erfindungsgemäßen festen, oral applizierbaren pharmazeutischen Darreichungsform mit modifizierter Freisetzung.

Weiterer Gegenstand der vorliegenden Erfindung ist ein Verfahren zur Prophylaxe, Sekundärprophylaxe und/oder Behandlung von arteriellen und/oder venösen thromboembolischen Erkrankungen durch Verabreichung einer erfindungsgemäßen festen, oral applizierbaren, den Wirkstoff (I) enthaltende pharmazeutischen Darreichungsform mit modifizierter Freisetzung.

Die Erfindung wird nachstehend durch bevorzugte Ausführungsbeispiele näher erläutert, auf welche sie jedoch nicht eingeschränkt ist. Soweit nicht anders angegeben, beziehen sich nachstehend alle Mengenangaben auf Gewichtsprozente.

### Experimenteller Teil

Soweit nicht anders angegeben, werden die im folgenden beschriebenen in vitro Freisetzungsuntersuchungen gemäß USP-Freisetzungsmethode mit Apparatur 2 (Paddle) durchgeführt. Die Umdrehungsgeschwindigkeit des Rührers liegt bei 75 UpM (Umdrehungen pro Minute) in 900 ml einer Pufferlösung von pH 6,8, die hergestellt wurde aus 1,25 ml ortho Phosphorsäure, 4.75 g Citronensäuremonohydrat und 27,46 g Dinatriumhydrogenphosphatdihydrat in 10 1 Wasser. Gegebenenfalls wird der Lösung noch < 1% Tensid, vorzugsweise Natriumlaurylsulfat zugegeben. Tablettenformulierungen werden vorzugsweise aus einem sinker, entsprechend der japanischen Pharmacopoeia freigesetzt.

### 1. Tablettenformulierungen basierend auf Erosionsmatrix-Systemen

### 1.1 Erosionsmatrixtablette enthaltend kristallinen Wirkstoff (I)

### Beispielformulierung 1.1.1

### Tablettenzusammensetzung in mg/Tablette

| | |
|---|---|
| Wirkstoff (I), mikronisiert | 25.0 mg |
| Mikrokristalline Cellulose | 10.0 mg |
| Laktose Monohydrat | 26.9 mg |
| Hydroxypropylcellulose, Typ HPC-L (Nisso) | 52.0 mg |
| Hydroxypropylcellulose, Typ HPC-M (Nisso) | 10.0 mg |
| Natriumlaurylsulfat | 0.5 mg |
| Magnesiumstearat | 0.6 mg |
| Hydroxypropylmethylcellulose, 15 cp | 1.8 mg |
| Polyethylenglycol 3.350 | 0.6 mg |
| Titandioxid | 0.6 mg |
| | 128.0 mg |

### Herstellung:

Eine Teilmenge Hydroxypropylcellulose Typ L und Natriumlaurylsulfat werden in Wasser gelöst. In diese Lösung wird der mikronisierte Wirkstoff (I) suspendiert. Die so hergestellte Suspension wird als Granulierflüssigkeit im Rahmen einer Wirbelschichtgranulation auf die Vorlage aus mikrokristalliner Cellulose, HPC-L und HPC-M und Laktose Monohydrat aufgesprüht. Nach Trocknung und Siebung (0.8 mm Maschenweite) des entstandenen Granulates wird Magnesiumstearat zugegeben und gemischt. Die so erhaltene pressfertige Mischung wird zu Tabletten mit 7 mm Durchmesser und einer Bruchfestigkeit von 50 bis 100 N verpresst. Die anschließende Lackierung der Tabletten erfolgt mit Titandioxid, das in einer wässrigen Lösung aus Hydroxypropylmethylcellulose (15 cp) und Polyethylenglycol suspendiert ist.

### Beispielformulierung 1.1.2

### Tablettenzusammensetzung in mg/Tablette

| | |
|---|---|
| Wirkstoff (I), mikronisiert | 25.0 mg |
| Mikrokristalline Cellulose | 10.0 mg |
| Laktose Monohydrat | 26.9 mg |
| Hydroxypropylcellulose, Typ HPC-L (Nisso) | 12.0 mg |
| Hydroxypropylcellulose, Typ HPC-M (Nisso) | 50.0 mg |
| Natriumlaurylsulfat | 0.5 mg |
| Magnesiumstearat | 0.6 mg |
| Hydroxypropylmethylcellulose, 15 cp | 1.8 mg |
| Polyethylenglycol 3.350 | 0.6 mg |
| Titandioxid | 0.6 mg |
| | 128.0 mg |

### Die Herstellung erfolgt analog der Beispielformulierung 1.1.1

### In vitro Freisetzung der Beipielformulierungen 1.1.1 und 1.1.2:

| | | | | | | |
|---|---|---|---|---|---|---|
| **Zeit [min]** | | 120 | 240 | 480 | 720 | 960 |
| **Freisetzung [%]** | **1.1.1** | 38 | 74 | 94 | 96 | 97 |
| | **1.1.2** | 14 | 32 | 66 | 89 | 98 |

### Methode: USP-Paddle, 75 UpM, 900 ml Phosphat-Puffer pH 6.8 + 0.5 % Natriumlaurylsulfat, JP-sinker

### 1.2 Erosionsmatrixtablette enthaltend amorphen Wirkstoff (I)

### Beispielformulierung 1.2

### Tablettenzusammensetzung in mg/Tablette

### Schmelzextrudat:

| | |
|---|---|
| Wirkstoff (I), mikronisiert | 30.0 mg |
| Hydroxypropylcellulose, Typ HPC-M (Nisso) | 210.0 mg |
| Xylitol | 60.0 mg |
| | 300.0 mg |

| **Tabletten:** | **A** | **B** | **C** |
|---|---|---|---|
| Schmelzextrudat, gemahlen | 300.0 mg | 300.0 mg | 300.0 mg |
| Mannitol (Pearlitol, Roquette) | 195.0 mg | 100.0 mg | ----- |
| Hydroxypropylcellulose (Typ HPC-L, Nisso) | ---- | ----- | 95.0 mg |
| Hydroxypropylmethycellulose (15 cp) | ----- | 95.0 mg | ----- |
| Mikrokristalline Cellulose | 50.0 mg | ----- | ----- |
| Hochdisperses Siliziumdioxid | 2.5 mg | 2.5 mg | 2.5 mg |
| (Aerosil 200, Degussa) | | | |
| Magnesiumstearat | 2.5 mg | 2.5 mg | 2.5 mg |
| | 550.0 mg | 500.0 mg | 400.0 mg |

### Herstellung:

Mikronisierter Wirkstoff (I), Hydroxypropylcellulose und Xylitol werden gemischt und in einem Doppelschneckenextruder (Leistritz Micro 18 PH) mit einer Austrittsdüse von 2 mm Durchmesser verarbeitet. Die Mischung wird bei einer Temperatur von 195°C extrudiert (gemessen am Düsenaustritt). Der erhaltene Extrudatstrang wird in 1 bis 2 mm große Stücke geschnitten und anschließend in einer Prallmühle gemahlen.

Nach Siebung (0.63 mm) werden die weiteren Hilfsstoffe (siehe obige Tabelle) dem gemahlenen Extrudat zugemischt und diese Mischung zu Tabletten im Oblongformat 15 x 7 mm (A + B) bzw. 14 x 7 mm (C) verpresst.

### In vitro Freisetzung der Formulierungen 1.2 A bis C:

| | | | | | |
|---|---|---|---|---|---|
| **Zeit [min]** | | 240 | 480 | 720 | 1440 |
| **Freisetzung [%]** | **A** | 30 | 63 | 83 | 95 |
| | **B** | 27 | 56 | 77 | 99 |
| | **C** | 23 | 45 | 64 | 98 |

### Methode: USP-Paddle, 75 UpM, 900 ml Phosphat-Puffer pH 6.8, JP-sinker

Eine konventionelle, schnell freisetzende Tablette, in der die gleiche Wirkstoffmenge von 30 mg Wirkstoff (I) pro Tablette in mikronisierter kristalliner Form vorliegt, erzielt unter gleichen Bedingungen nur eine unvollständige Wirkstofffreisetzung: Nach 4 bis 6 Stunden ist dort ein Plateau mit lediglich ca. 33 % Wirkstofffreisetzung erreicht. Im Vergleich dazu zeigt die praktisch vollständige Wirkstofffreisetzung der Extrudatformulierungen A-C in dem tensidfreien Freisetzungsmedium eine sehr deutliche Erhöhung der Löslichkeit des Wirkstoffes (I). Dieses konnte durch die Überführung des Wirkstoffes (I) in den amorphen Zustand mittels Schmelzextrusionsverfahren erreicht werden.

### 2. Multipartikuläre Zubereitungen

### 2.1 Mini-Tabletten enthaltend kristallinen Wirkstoff (I)

### Beispielformulierung 2.1

### Tablettenzusammensetzung in mg/Minitablette

| | |
|---|---|
| Wirkstoff (I), mikronisiert | 0.50 mg |
| Hydroxypropylcellulose (Klucel HXF, Hercules) | 5.91 mg |
| Hydroxypropylcellulose (HPC-L, Nisso) | 0.04 mg |
| Natriumlaurylsulfat | 0.01 mg |
| Magnesiumstearat | 0.04 mg |
| | 6.50 mg |

### Herstellung:

Hydroxypropylcellulose Klucel HXF wird mit einer wässrigen Suspension von Wirkstoff (I) und Hydroxypropylcellulose Typ HPC-L und Natriumlaurylsulfat granuliert. Nach Trocknung und Siebung des entstandenen Granulates wird Magnesiumstearat zugegeben und gemischt. Die so erhaltene pressfertige Mischung wird zu 2 mm-Minitabletten von 6.5 mg verpresst. Die Freisetzung einer zu 25 mg Wirkstoff (I) äquivalenten Menge der Minitabletten (50 Stück) ist nachfolgend aufgeführt:

### in vitro Freisetzung der Formulierung 2.1:

| | | | | |
|---|---|---|---|---|
| **Zeit [min]** | 240 | 480 | 720 | 1200 |
| **Freisetzung [%]** | 14 | 31 | 52 | 89 |

### Methode: USP-Paddle, 75 UpM, 900 ml Phosphat-Puffer pH 6.8 + 0.5 % Natriumlaurylsulfat

### 2.2 Pellets enthaltend amorphen Wirkstoff (I)

### Beispielformulierung 2.2.1

### Zusammensetzung in mg pro 30 mg Einzeldosis Wirkstoff (I)

### Schmelzextrudat

| | |
|---|---|
| Wirkstoff (I), mikronisiert | 30.0 mg |
| Hydroxypropylcellulose, Typ Klucel HXF (Hercules) | 510.0 mg |
| Xylitol | 60.0 mg |
| | 600.0 mg |

### Lackhülle

| | |
|---|---|
| Hydroxypropylmethylcellulose, 3 cp | 15.0 mg |
| Magnesiumstearat | 6.9 mg |
| Poly(ethylacrylat, methylmethacrylat)-Dispersion 30 % | 126.0 mg * |
| (Eudragit NE 30 D, Röhm/Degussa) | |
| Polysorbat 20 | 0.3 mg |
| | 60.0 mg ** |

| | |
|---|---|
| *entspricht 37.8 mg Lacktr-ockensubstan **Lacktrockensubstanz | |

### Herstellung:

Mikronisierter Wirkstoff (J), Hydroxypropylcellulose und Xylitol werden gemischt. 1.5 kg dieser Mischung werden in einem Doppelschneckenextruder (Leistritz Micro 18 PH) mit einer Austrittsdüse von 2 mm Durchmesser verarbeitet. Die Mischung wird bei einer Temperatur von 200°C extrudiert (gemessen am Düsenaustritt). Der erhaltene Extrudatstrang wird in 1,5 mm große Stucke geschnitten. Nach Siebung zur Abtrennung des Feinanteils werden die Pellets in der Wirbelschicht lackiert. Dazu wird eine wässrige Lackdispersion bestehend aus oben beschriebenen Komponenten und 20 %igem Feststoffanteil auf die Partikel gesprüht. Nach Trocknung und Siebung kann die Abfüllung der Pellets beispielsweise in Gläser, Sachetbeutel oder Hartgelatinekapseln erfolgen.

### Beispielformulierung 2.2.2

### Zusammensetzung in mg pro 30 mg Einzeldosis Wirkstoff (I)

### Schmelzextrudat

| | |
|---|---|
| Wirkstoff (I), mikronisiert | 30.0 mg |
| Hydroxypropylcellulose, Typ Klucel HXF (Hercules) | 570.0 mg |
| | 600.0 mg |

### Lackhülle

| | |
|---|---|
| Hydroxypropylmethylcellulose, 3 cp | 15.0 mg |
| Magnesiumstearat | 6.9 mg |
| Poly(ethylacrylat, methylmethacrylat)-Dispersion 30 % | 126.0 mg * |
| (Eudragit NE 30 D, Röhm/Degussa) | |
| Polysorbat 20 | 0.3 mg |
| | 60.0 mg ** |

| | |
|---|---|
| *entspricht 37.8 mg Lacktrockensubstanz **Lacktrockensubstanz | |

### Herstellung: analog 2.2.1

Ein ähnliches Vorgehen/Verfahren zur Herstellung multipartikulärer Retardzubereitungen ist zwar in EP 1 113 787 beschrieben, allerdings mit dem Unterschied, dass in den hier beschriebenen Beispielen 2.2.1 und 2.2.2 der Wirkstoff (I) aufgrund geeigneter Prozessparameter in die amorphe Form überführt wird. Dadurch wird insbesondere eine Erhöhung der Wirkstofflöslichkeit erreicht:

### in vitro Freisetzung der Formulierungen 2.2.1 und 2.2.2

| | | | | | |
|---|---|---|---|---|---|
| **Zeit [min]** | | 240 | 480 | 720 | 1440 |
| **Freisetzung [%]** | **3.2.1** | 34 | 69 | 91 | 95 |
| | **3.2.2** | 30 | 57 | 80 | 94 |

### Methode: USP-Paddle, 75 UpM, 900 ml Phosphat-Puffer pH 6.8

Darreichungsformen enthaltend den Wirkstoff (I) in kristalliner Form erzielen unter gleichen Bedingungen nur eine Freisetzung von ca. 33 % (siehe auch die Diskussion bei den Freisetzungsergebnissen der Beispielformulierung 1.2)

### 3. Osmotische Systeme

### 3.1 Einkammersystem enthaltend kristallinen Wirkstoff (I)

### Beispielformulierung 3.1

### Tablettenzusammensetzung in mg/Tablette (deklarierter Gehalt = 30 mg/Tablette)

### Kern

| | |
|---|---|
| Wirkstoff (I), mikronisiert | 36.0 mg |
| Xanthangummi (Rhodigel TSC, Rhodia) | 100.0 mg |
| Copolyvidon (Kollidon VA 64, BASF) | 55.0 mg |
| Natriumchlorid | 55.0 mg |
| Natriumbicarbonat | 17.5 mg |
| Natriumcarboxymethylstärke | 23.0 mg |
| Hydroxypropylmethylcellulose (5 cp) | 10.0 mg |
| Natriumlaurylsulfat | 0.5 mg |
| Hochdisperses Siliziumdioxid (Aerosil 200, Degussa) | 1.5 mg |
| Magnesiumstearat | 1.5 mg |
| | 300.0 mg |

### Hülle (osmotische Membran)

| | |
|---|---|
| Celluloseacetat | 19.95 mg |
| Polyethylenglycol 400 | 1.05 mg |
| | 21.00 mg |

### Herstellung:

Xanthangumm, Copolyvidon, Natriumchlorid, Natriumbicarbonat und Natriumcarboxy-methylcellulose werden gemischt und anschließend mit einer wässrigen Suspension aus Wirkstoff (I) und Hydroxypropylmethylcellulose feucht granuliert. Nach Trocknung und Siebung werden Aerosil und Magnesiumstearat zugemischt und die so erhaltene pressfertige Mischung zu Tabletten mit 8 mm Durchmesser verpresst. Die Tablettenkerne werden mit einer acetonischen Lösung von Celluloseacetat und Polyethylenglycol beschichtet und getrocknet. Anschließend werden bei jeder Tablette zwei Öffnungen von je 1 mm Durchmesser mittels eines Handbohrers angebracht.

### In vitro Freisetzung der Beispielformulierung 3.1

| | | | | |
|---|---|---|---|---|
| **Zeit [min]** | 240 | 480 | 720 | 1440 |
| **Freisetzung [%]** | 21 | 54 | 72 | 90 |

### Methode: USP-Paddle, 100 UpM, 900 ml Phosphat-Puffer pH 6.8 + 1.0 % Natriumlaurylsulfat, JP-sinker

### 3.2 Zweikammersystem enthaltend kristallinen Wirkstoff (I)

### Beispielformulierung 3.2

### Tablettenzusammensetzung in mg/Tablette (deklarierter Gehalt = 30 mg/Tablette)

### Kern

### Wirkstoffschicht

| | |
|---|---|
| Wirkstoff (I), mikronisiert | 33.0 mg |
| Hydroxypropylmethylcellulose (5 cp) | 8.2 mg |
| Polyethylenoxid * | 122.2 mg |
| Hochdisperses Siliziumdioxid (Aerosil 200, Degussa) | 1.3 mg |
| Magnesiumstearat | 0.8 mg |
| | 165.5 mg |

### Osmoseschicht

| | |
|---|---|
| Hydroxypropylmethylcellulose (5 cp) | 4.1 mg |
| Natriumchlorid | 23.9 mg |
| Polyethylenoxid ** | 52.9 mg |
| Eisenoxid rot | 0.8 mg |
| Magnesiumstearat | 0.2 mg |
| | 81.9mag |

### Hülle (osmotische Membran)

| | |
|---|---|
| Celluloseacetat | 29.07 mg |
| Polyethylenglycol 400 | 1.53mg |
| | 30.60 mg |

| | |
|---|---|
| * Viskosität 5 %ige wässrige Lösung (25°C, Brookfield Viskosimeter Modell RVT, Spindel Nr. 1, Drehzahl: 50 U/min): 40-100 mPa·s (z. B. POLYOX™ Water-Soluble Resin NF WSR N-80; Dow) ** Viskosität 1 %ige wässrige Lösung (25°C, Brookfield Viskosimeter Modell RVF, Spindel Nr. 2, Drehzahl: 2 U/min): 5000-8000 mPa-s (z. B. POLYOX™ Water-Soluble Resin NF WSR Coagulant; Dow) | |

### Herstellung:

Die Komponenten der Wirkstoffschicht werden gemischt und trocken granuliert (Walzengranulation). Ebenso werden die Komponenten der Osmoseschicht gemischt und trocken granuliert (Walzengranulation). Auf einer Zweischichttablettenpresse werden beide Granulate zu einer Zweischichttablette (Durchmesser 8.7 mm) verpresst. Die Tabletten werden mit einer acetonischen Lösung von Celluloseacetat und Polyethylenglycol beschichtet und getrocknet. Anschließend wird bei jeder Tablette auf der Wirkstoffseite eine Öffnung von 0.9 mm Durchmesser mittels eines Handbohrers angebracht.

### In vitro Freisetzung der Beispielformulierung 3.2

| | | | | |
|---|---|---|---|---|
| **Zeit [min]** | 240 | 480 | 720 | 1200 |
| **Freisetzung [%]** | 21 | 54 | 81 | 99 |

Methode: USP-Paddle, 100 UpM, 900 ml Phosphat-Puffer pH 6.8 + 1.0 % Natriumlaurylsulfat, JP-sinker

## Patentansprüche

1. Feste, oral applizierbare, 5-Chlor-*N*-({(5*S*)-2-oxo-3-[4-(3-oxo-4-morpholinyl)-phenyl]-1,3-oxazolidin-5-yl}-methyl)-2-thiophencarboxamid (I) enthaltende pharmazeutische Darreichungsform mit modifizierter Freisetzung, **dadurch gekennzeichnet, dass** 80 % des Wirkstoffes (I) in einem Zeitraum von 2 bis 24 Stunden gemäß USP-Freisetzungsmethode mit Apparatur 2 (Paddle) freigesetzt werden.

2. Pharmazeutische Darreichungsform gemäß Anspruch 1, **dadurch gekennzeichnet, dass** 80 % des Wirkstoffes (I) in einem Zeitraum von 4 bis 20 Stunden gemäß USP-Freisetzungsmethode mit Apparatur 2 (Paddle) freigesetzt werden.

3. Pharmazeutische Darreichungsform gemäß Anspruch 1 oder 2, **dadurch gekennzeichnet, dass** der Wirkstoff (I) in kristalliner Form vorliegt.

4. Pharmazeutische Darreichungsform gemäß Anspruch 3, enthaltend den Wirkstoff (I) in mikronisierter Form.

5. Pharmazeutische Darreichungsform gemäß Anspruch 4, enthaltend den Wirkstoff (I) in hydrophylisierter Form.

6. Pharmazeutische Darreichungsform gemäß Anspruch 1 oder 2, **dadurch gekennzeichnet, dass** der Wirkstoff (I) in amorpher Form vorliegt.

7. Pharmazeutische Darreichungsform gemäß Anspruch 6, **dadurch gekennzeichnet, dass** der Wirkstoff (I) über Schmelzextrusion amorphisiert wurde.

8. Pharmazeutische Darreichungsform gemäß Anspruch 7, **dadurch gekennzeichnet, dass** als Polymer bei der Schmelzextrusion Hydroxypropylcellulose (HPC) oder Polyvinylpyrrolidon (PVP) eingesetzt wird, der Polymer-Anteil im Schmelzextrudat mindestens 50 % beträgt und der Wirkstoff (I) im Schmelzextrudat in einer Konzentration von 1 bis 20 % vorliegt.

9. Pharmazeutische Darreichungsform gemäß einem der Ansprüche 7 oder 8, **dadurch gekennzeichnet, dass** mindestens ein pharmazeutisch geeigneter Stoff in einer Konzentration von 2 bis 40 % als Weichmacher und/oder zur Erniedrigung der Schmelztemperatur des Wirkstoffes (I) zugesetzt wird.

10. Pharmazeutische Darreichungsform gemäß Anspruch 9, **dadurch gekennzeichnet, dass** der pharmazeutisch geeignete Zusatzstoff ein Zuckeralkohol ist.

11. Pharmazeutische Darreichungsform gemäß einem der Ansprüche 1 oder 2, basierend auf einem Erosionsmatrix-System.

12. Pharmazeutische Darreichungsform gemäß Anspruch 11, **dadurch gekennzeichnet, dass** der Wirkstoff (I) in amorpher Form vorliegt.

13. Pharmazeutische Darreichungsform gemäß einem der Ansprüche 11 oder 12, enthaltend Hydroxypropylcellulose oder Hydroxypropylmethylcellulose oder Gemische von Hydroxypropylcellulose und Hydroxypropylcellulose als hydrophilen Matrixbildner.

14. Pharmazeutische Darreichungsform gemäß einem der Ansprüche 11 bis 13, **dadurch gekennzeichnet, dass** der Wirkstoff (I) in einer Konzentration zwischen 1 und 50 % enthalten ist.

15. Verfahren zur Herstellung einer pharmazeutische Darreichungsform gemäß einem der Ansprüche 11 bis 14, **dadurch gekennzeichnet, dass** mit Hilfe der Schmelzextrusion ein den Wirkstoff (I) enthaltendes Extrudat hergestellt wird, welches gemahlen, mit weiteren Tablettierhilfsstoffen gemischt und anschließend mittels Direkttablettierung zu Tabletten verpresst wird.

16. Multipartikuläre pharmazeutische Darreichungsform gemäß einem der Ansprüche 1 oder 2.

17. Multipartikuläre pharmazeutische Darreichungsform gemäß Anspruch 16, **dadurch gekennzeichnet, dass** der Wirkstoff (I) in amorpher Form vorliegt.

18. Multipartikuläre pharmazeutische Darreichungsform gemäß einem der Ansprüche 16 oder 17, enthaltend Hydroxypropylcellulose als hydrophilen Matrixbildner.

19. Multipartikuläre pharmazeutische Darreichungsform gemäß Anspruch 18, **dadurch gekennzeichnet, dass** Hydroxypropylcellulose als hydrophiler Matrixbildner in einer Konzentration zwischen 10 und 99 % enthalten ist.

20. Multipartikuläre pharmazeutische Darreichungsform gemäß einem der Ansprüche 16 bis 19, **dadurch gekennzeichnet, dass** der Wirkstoff (I) in einer Konzentration zwischen 1 und 30 % enthalten ist.

21. Multipartikuläre pharmazeutische Darreichungsform gemäß einem der Ansprüche 16 bis 20, **dadurch gekennzeichnet, dass** der Durchmesser der Partikel zwischen 0,5 und 3,0 mm beträgt.

22. Multipartikuläre pharmazeutische Darreichungsform gemäß Anspruch 21, **dadurch gekennzeichnet, dass** der Durchmesser der Partikel zwischen 1,0 und 2,5 mm beträgt.

23. Pharmazeutische Darreichungsform enthaltend multipartikuläre pharmazeutische Darreichungsformen gemäß einem der Ansprüche 16 bis 22.

24. Pharmazeutische Darreichungsform gemäß Anspruch 23 in Form einer Kapsel, eines Sachets oder einer Tablette.

25. Verfahren zur Herstellung einer multipartikulären pharmazeutischen Darreichungsform, wie in einem der Ansprüche 16 bis 22 definiert, **dadurch gekennzeichnet, dass** durch Schmelzextrusion ein den Wirkstoff (I) enthaltender Extrudatstrang hergestellt wird, der geschnitten wird.

26. Verfahren gemäß Anspruch 25, **dadurch gekennzeichnet, dass** die nach Schneiden des Extrudatstranges erhaltenen Formkörper ausgerundet werden.

27. Verfahren gemäß einem der Ansprüche 25 oder 26, **dadurch gekennzeichnet, dass** die erhaltenen Formkörper lackiert werden.

28. Pharmazeutische Darreichungsform gemäß einem der Ansprüche 1 oder 2, basierend auf einem osmotische Freisetzungssystem.

29. Pharmazeutische Darreichungsform gemäß Anspruch 28, **dadurch gekennzeichnet, dass** der Wirkstoff (I) in amorpher Form vorliegt.

30. Pharmazeutische Darreichungsform gemäß einem der Ansprüche 28 oder 29, bestehend aus einem osmotischen Einkammersystem umfassend einen Kern, enthaltend
• 2 bis 30 % Wirkstoff (I),
• 20 bis 50 % Xanthan,
• 10 bis 30 % eines Vinylpyrrolidon-Vinylacetat-Copolymers,
sowie eine Hülle, bestehend aus einem wasserdurchlässigen, für die Komponenten des Kerns undurchlässigen Material mit mindestens einer Öffnung.

31. Pharmazeutische Darreichungsform gemäß Anspruch 30, die im Kern zusätzlich Natriumchlorid als osmotisch aktiven Zusatz enthält.

32. Pharmazeutische Darreichungsform gemäß einem der Ansprüche 30 oder 31, **dadurch gekennzeichnet, dass** die Hülle aus Celluloseacetat oder einem Gemisch von Celluloseacetat und Polyethylenglycol besteht.

33. Verfahren zur Herstellung eines osmotischen Einkammersystems, wie in einem der Ansprüche 30 bis 32 definiert, **dadurch gekennzeichnet, dass** die Komponenten des Kerns miteinander vermischt, granuliert und tablettiert werden, der so entstandene Kern mit einer Hülle beschichtet wird und die Hülle abschließend mit einer oder mehreren Öffnungen versehen wird.

34. Pharmazeutische Darreichungsform gemäß einem der Ansprüche 28 oder 29, bestehend aus einem osmotischen Zweikammersystem umfassend einen Kern mit einer Wirkstoffschicht, enthaltend
• 1 bis 40 % Wirkstoff (I),
• 50 bis 95 % von einem oder mehreren osmotisch aktiven Polymeren,
und eine Osmoseschicht, enthaltend
• 40 bis 90 % von einem oder mehreren osmotisch aktiven Polymeren
• 10 bis 40 % eines osmotisch aktiven Zusatzes,
sowie eine Hülle, bestehend aus einem wasserdurchlässigen, für die Komponenten des Kerns undurchlässigen Material mit mindestens einer Öffnung

35. Pharmazeutische Darreichungsform gemäß Anspruch 34, die im Kern in der Wirkstoffschicht olyethylenoxid mit einer Viskosität von 40 bis 100 mPa · s (5 %ige wässrige Lösung, 25°C) als osmotisch aktives Polymer enthält und im Kern in der Osmoseschicht Polyethylenoxid mit einer Viskosität von 5000 bis 8000 mPa · s (1 %ige wässrige Lösung, 25°C) als osmotisch aktives Polymer enthält.

36. Pharmazeutische Darreichungsform gemäß einem der Ansprüche 34 oder 35, **dadurch gekennzeichnet, dass** die Hülle aus Celluloseacetat oder einem Gemisch von Celluloseacetat und Polyethylenglycol besteht.

37. Verfahren zur Herstellung eines osmotischen Zweikammersystems, wie in einem der Ansprüche 34 bis 36 definiert, **dadurch gekennzeichnet, dass**
• die Komponenten der Wirkstoffschicht gemischt und granuliert werden und
• die Komponenten der Osmoseschicht gemischt und granuliert werden,
• anschließend auf einer Zweischichttablettenpresse beide Granulate zu einer Zweischichttablette verpresst werden,
• der so entstandene Kern dann mit der Hülle beschichtet wird und
• die Hülle auf der Wirkstoffseite mit einer oder mehreren Öffnungen ersehen wird.

38. Arzneimittel, enthaltend eine feste, oral applizierbare pharmazeutische Darreichungsform mit einer modifizierten, wie in Anspruch 1 definierten Freisetzung des Wirkstoffes (I).

39. Verwendung einer festen, oral applizierbaren, den Wirkstoff (I) enthaltende pharmazeutischen Darreichungsform mit modifizierter Freisetzung, wie in Anspruch 1 definiert, zur Herstellung eines Arzneimittels zur Prophylaxe, Sekundärprophylaxe und/oder Behandlung von Erkrankungen.

40. Verwendung nach Anspruch 39 zur Herstellung eines Arzneimittels zur Prophylaxe, Sekundärprophylaxe und/oder Behandlung von thromboembolischen Erkrankungen.

41. Verwendung nach Anspruch 40 zur Herstellung eines Arzneimittels zur Prophylaxe, Sekundärprophylaxe und/oder Behandlung von Myocardinfarkt, Angina Pectoris, Reokklusionen und Restenosen nach einer Angioplastie oder aortokoronarem Bypass, Schlaganfall, transitorische ischämische Attacken, periphere arterielle Verschlusskrankheiten, Lungenembolien oder tiefen Venenthrombosen.

42. Verwendung von 5-Chlor-*N-*({(5*S*)-2-oxo-3-[4-(3-oxo-4-morpholinyl)-phenyl]-1,3-oxazolidin-5-yl}-methyl)-2-thiophencarboxamid (I) zur Herstellung einer pharmazeutischen Darreichungsform, wie in Anspruch 1 definiert.

## Claims

1. Solid, modified-release pharmaceutical dosage form which can be administered orally and comprises 5-chloro-*N*-({(5*S*)-2-oxo-3-[4-(3-oxo-4-morpholinyl)phenyl]-1,3-oxazolidin-5-yl}methyl)-2-thiophenecarboxamide (I), **characterized in that** 80% of the active ingredient (I) are released in a period of from 2 to 24 hours in the USP release method with apparatus 2 (paddle).

2. Pharmaceutical dosage form according to Claim 1, **characterized in that** 80% of the active ingredient (I) are released in a period of from 4 to 20 hours in the USP release method with apparatus 2 (paddle).

3. Pharmaceutical dosage form according to Claim 1 or 2, **characterized in that** the active ingredient (I) is present in crystalline form.

4. Pharmaceutical dosage form according to Claim 3, comprising the active ingredient (I) in micronized form.

5. Pharmaceutical dosage form according to Claim 4, comprising the active ingredient (I) in hydrophylized form.

6. Pharmaceutical dosage form according to Claim 1 or 2, **characterized in that** the active ingredient (I) is present in amorphous form.

7. Pharmaceutical dosage form according to Claim 6, **characterized in that** the active ingredient (I) has been amorphized by melt extrusion.

8. Pharmaceutical dosage form according to Claim 7, **characterized in that** the polymer employed in the melt extrusion is hydroxypropylcellulose (HPC) or polyvinylpyrrolidone (PVP), the proportion of polymer in the melt extrudate is at least 50%, and the active ingredient (I) is present in the melt extrudate in a concentration of from 1 to 20%.

9. Pharmaceutical dosage form according to either of Claims 7 or 8, **characterized in that** at least one pharmaceutically suitable substance is added in a concentration of from 2 to 40% as plasticizer and/or to depress the melting point of the active ingredient (I).

10. Pharmaceutical dosage form according to Claim 9, **characterized in that** the pharmaceutically suitable additive is a sugar alcohol.

11. Pharmaceutical dosage form according to either of Claims 1 or 2, based on an erosion matrix system.

12. Pharmaceutical dosage form according to Claim 11, **characterized in that** the active ingredient (I) is present in amorphous form.

13. Pharmaceutical dosage form according to either of Claims 11 or 12, comprising hydroxypropylcellulose or hydroxypropylmethylcellulose or mixtures of hydroxypropylcellulose and hydroxypropylmethylcellulose as hydrophilic matrix former.

14. Pharmaceutical dosage form according to any of Claims 11 to 13, **characterized in that** the active ingredient (I) is present in a concentration of between 1 and 50%.

15. Process for producing a pharmaceutical dosage form according to any of Claims 11 to 14, **characterized in that** an extrudate comprising the active ingredient (I) is produced by melt extrusion and is ground, mixed with further tabletting excipients and then compressed to tablets by direct tabletting.

16. Multiparticulate pharmaceutical dosage form according to either of Claims 1 or 2.

17. Multiparticulate pharmaceutical dosage form according to Claim 16, **characterized in that** the active ingredient (I) is present in amorphous form.

18. Multiparticulate pharmaceutical dosage form according to either of Claims 16 or 17, comprising hydroxypropylcellulose as hydrophilic matrix former.

19. Multiparticulate pharmaceutical dosage form according to Claim 18, **characterized in that** hydroxypropylcellulose is present as hydrophilic matrix former in a concentration of between 10 and 99%.

20. Multiparticulate pharmaceutical dosage form according to any of Claims 16 to 19, **characterized in that** the active ingredient (I) is present in a concentration of between 1 and 30%.

21. Multiparticulate pharmaceutical dosage form according to any of Claims 16 to 20, **characterized in that** the diameter of the particles is between 0.5 and 3.0 mm.

22. Multiparticulate pharmaceutical dosage form according to Claim 21, **characterized in that** the diameter of the particles is between 1.0 and 2.5 mm.

23. Pharmaceutical dosage form comprising multiparticulate pharmaceutical dosage forms according to any of Claims 16 to 22.

24. Pharmaceutical dosage form according to Claim 23 in the form of a capsule, of a sachet or of a tablet.

25. Process for producing a multiparticulate pharmaceutical dosage form as defined in any of Claims 16 to 22, **characterized in that** an extrudate strand comprising the active ingredient (I) is produced by melt extrusion and is cut.

26. Process according to Claim 25, **characterized in that** the articles obtained after cutting the extrudate strand are rounded.

27. Process according to either of Claims 25 or 26, **characterized in that** the resulting articles are coated.

28. Pharmaceutical dosage form according to either of Claims 1 or 2, based on an osmotic release system.

29. Pharmaceutical dosage form according to Claim 28, **characterized in that** the active ingredient (I) is present in amorphous form.

30. Pharmaceutical dosage form according to either of Claims 28 or 29, consisting of an osmotic single-chamber system comprising a core comprising
• 2 to 30% active ingredient (I),
• 20 to 50% xanthan,
• 10 to 30% of a vinylpyrrolidone-vinyl acetate copolymer,
and a shell consisting of a water-permeable material which is impermeable for the components of the core and has at least one orifice.

31. Pharmaceutical dosage form according to Claim 30, which additionally comprises sodium chloride as osmotically active additive in the core.

32. Pharmaceutical dosage form according to either of Claims 30 or 31, **characterized in that** the shell consists of cellulose acetate or of a mixture of cellulose acetate and polyethylene glycol.

33. Process for producing an osmotic single-chamber system as defined in any of Claims 30 to 32, **characterized in that** the components of the core are mixed together, granulated and tabletted, the core produced in this way is coated with a shell, and the shell is finally provided with one or more orifices.

34. Pharmaceutical dosage form according to either of Claims 28 or 29, consisting of an osmotic two-chamber system comprising a core having an active ingredient layer comprising
• 1 to 40% active ingredient (I),
• 50 to 95% of one or more osmotically active polymers,
and an osmosis layer comprising
• 40 to 90% of one or more osmotically active polymers,
• 10 to 40% of an osmotically active addition,
and a shell consisting of a water-permeable material which is impermeable for the components of the core and has at least one orifice.

35. Pharmaceutical dosage form according to Claim 34, which comprises polyethylene oxide having a viscosity of from 40 to 100 mPa · s (5% strength aqueous solution, 25°C) as osmotically active polymer in the active ingredient layer in the core, and comprises polyethylene oxide having a viscosity of from 5000 to 8000 mPa · s (1% strength aqueous solution, 25°C) as osmotically active polymer in the osmosis layer in the core.

36. Pharmaceutical dosage form according to either of Claims 34 or 35, **characterized in that** the shell consists of cellulose acetate or of a mixture of cellulose acetate and polyethylene glycol.

37. Process for producing an osmotic two-chamber system as defined in any of Claims 34 to 36, **characterized in that**
• the components of the active ingredient layer are mixed and granulated and
• the components of the osmosis layer are mixed and granulated,
• subsequently the two granules are compressed in a bilayer tablet press to a bilayer tablet.
• the core produced in this way is then coated with the shell, and
• the shell is provided with one or more orifices on the active ingredient side.

38. Medicament comprising a solid pharmaceutical dosage form which can be administered orally and has a modified release, as defined in Claim 1, of the active ingredient (I).

39. Use of a solid pharmaceutical dosage form which can be administered orally and comprises the active ingredient (I) and has modified release as defined in Claim 1 for producing a medicament for the prophylaxis, secondary prophylaxis and/or treatment of disorders.

40. Use according to Claim 39 for producing a medicament for the prophylaxis, secondary prophylaxis and/or treatment of thromboembolic disorders.

41. Use according to Claim 40 for producing a medicament for the prophylaxis, secondary prophylaxis and/or treatment of myocardial infarction, angina pectoris, reocclusions and restenoses following an angioplasty or aortocoronary bypass, stroke, transient ischaemic attacks, peripheral arterial occlusive diseases, pulmonary embolisms or deep vein thromboses.

42. Use of 5-chloro-*N-*({(5*S*)-2-oxo-3-[4-(3-oxo-4-morpholinyl)phenyl]-1,3-oxazolidin-5-yl}methyl)-2-thiophenecarboxamide (I) for producing a pharmaceutical dosage form as defined in Claim 1.

## Revendications

1. Forme d'administration pharmaceutique contenant du 5-chloro-*N-(*{(5*S*)-2-oxo-3-[4-(3-oxo-4-morpholinyl)-phényl]-1,3-oxazolidin-5-yl}-méthyl)-2-thiophènecarboxamide (I), applicable par voie orale, solide, à libération modifiée, **caractérisée en ce que** 80 % du principe actif (I) sont libérés en une durée de 2 à 24 h selon la méthode de libération USP avec l'appareil 2 (paddle).

2. Forme d'administration pharmaceutique selon la revendication 1, **caractérisée en ce que** 80 % du principe actif (I) sont libérés en une durée de 4 à 20 h selon la méthode de libération USP avec l'appareil 2 (Paddle).

3. Forme d'administration pharmaceutique selon la revendication 1 ou 2, **caractérisée en ce que** le principe actif (I) est sous forme cristalline.

4. Forme d'administration pharmaceutique selon la revendication 3 contenant le principe actif (I) sous forme micronisée.

5. Forme d'administration pharmaceutique selon la revendication 4 contenant le principe actif (I) sous forme rendue hydrophile.

6. Forme d'administration pharmaceutique selon la revendication 1 ou 2, **caractérisée en ce que** le principe actif (I) est sous forme amorphe.

7. Forme d'administration pharmaceutique selon la revendication 6, **caractérisée en ce que** le principe actif (I) a été rendu amorphe par extrusion à l'état fondu.

8. Forme d'administration pharmaceutique selon la revendication 7, **caractérisée en ce que** de l'hydroxypropylcellulose (HPC) ou de la polyvinylpyrrolidone (PVP) est utilisée comme polymère lors de l'extrusion à l'état fondu, la proportion de polymère dans le produit d'extrusion à l'état fondu est d'au moins 50 % et le principe actif (I) est présent dans le produit d'extrusion à l'état fondu en une concentration de 1 à 20 %.

9. Forme d'administration pharmaceutique selon l'une des revendications 7 et 8, **caractérisée en ce qu'**au moins une substance appropriée du point de vue pharmaceutique est ajoutée en une concentration de 2 à 40 % comme plastifiant et/ou pour abaisser la température de fusion du principe actif (I).

10. Forme d'administration pharmaceutique selon la revendication 9, **caractérisée en ce que** l'additif approprié du point de vue pharmaceutique est un sucre-alcool.

11. Forme d'administration pharmaceutique selon l'une des revendications 1 et 2 à base d'un système à matrice d'érosion.

12. Forme d'administration pharmaceutique selon la revendication 11, **caractérisée en ce que** le principe actif (I) est sous forme amorphe.

13. Forme d'administration pharmaceutique selon l'une des revendications 11 et 12 contenant de l'hydroxypropylcellulose ou de l'hydroxypropylméthylcellulose ou des mélanges d'hydroxypropylcellulose et d'hydroxypropylméthylcellulose comme formateur de matrice hydrophile.

14. Forme d'administration pharmaceutique selon l'une des revendications 11 à 13, **caractérisée en ce que** le principe actif (I) est contenu en une concentration entre 1 et 50 %.

15. Procédé pour produire une forme d'administration pharmaceutique selon l'une des revendications 11 à 14, **caractérisé en ce qu'**à l'aide de l'extrusion à l'état fondu, un produit d'extrusion contenant le principe actif (I) est produit, lequel est broyé, mélangé avec d'autres adjuvants de production de comprimés puis compressé en comprimés par production directe de comprimés.

16. Forme d'administration pharmaceutique multiparticulaire selon l'une des revendications 1 et 2.

17. Forme d'administration pharmaceutique multiparticulaire selon la revendication 16, **caractérisée en ce que** le principe actif (I) est sous forme amorphe.

18. Forme d'administration pharmaceutique multiparticulaire selon l'une des revendications 16 et 17 contenant de l'hydroxypropylcellulose comme formateur de matrice hydrophile.

19. Forme d'administration pharmaceutique multiparticulaire selon la revendication 18, **caractérisée en ce que** l'hydroxypropylcellulose est contenue comme formateur de matrice hydrophile en une concentration entre 10 et 99 %.

20. Forme d'administration pharmaceutique multiparticulaire selon l'une des revendications 16 à 19, **caractérisée en ce que** le principe actif (I) est contenu en une concentration entre 1 et 30 %.

21. Forme d'administration pharmaceutique multiparticulaire selon l'une des revendications 16 à 20, **caractérisée en ce que** le diamètre des particules est entre 0,5 et 3,0 mm.

22. Forme d'administration pharmaceutique multiparticulaire selon la revendication 21, **caractérisée en ce que** le diamètre des particules est entre 1,0 et 2,5 mm.

23. Forme d'administration pharmaceutique contenant des formes d'administration pharmaceutiques multiparticulaires selon l'une des revendications 16 à 22.

24. Forme d'administration pharmaceutique selon la revendication 23 sous forme d'une capsule, d'un sachet ou d'un comprimé.

25. Procédé pour produire une forme d'administration pharmaceutique multiparticulaire, telle que définie dans l'une des revendications 16 à 22, **caractérisé en ce qu'**un jonc de produit d'extrusion contenant le principe actif (I) est produit par extrusion à l'état fondu puis coupé.

26. Procédé selon la revendication 25, **caractérisé en ce que** les corps mis en forme obtenus après la coupe du jonc de produit d'extrusion sont arrondis.

27. Procédé selon l'une des revendications 25 et 26, **caractérisé en ce que** les corps mis en forme obtenus sont laqués.

28. Forme d'administration pharmaceutique selon l'une des revendications 1 et 2 à base d'un système de libération osmotique.

29. Forme d'administration pharmaceutique selon la revendication 28, **caractérisée en ce que** le principe actif (I) est sous forme amorphe.

30. Forme d'administration pharmaceutique selon l'une des revendications 28 et 29 consistant en un système osmotique à une chambre comprenant un noyau, contenant
• 2 à 30 % de principe actif (I),
• 20 à 50 % de xanthane,
• 10 à 30 % d'un copolymère vinylpyrrolidone-acétate de vinyle,
ainsi qu'une enveloppe, consistant en un matériau perméable à l'eau, imperméable aux composants du noyau, avec au moins une ouverture.

31. Forme d'administration pharmaceutique selon la revendication 30 qui contient en outre dans le noyau du chlorure de sodium comme additif actif du point de vue osmotique.

32. Forme d'administration pharmaceutique selon l'une des revendications 30 et 31, **caractérisée en ce que** l'enveloppe consiste en acétate de cellulose ou en un mélange d'acétate de cellulose et de polyéthylèneglycol.

33. Procédé pour produire un système osmotique à une chambre, tel que défini dans l'une des revendications 30 à 32, **caractérisé en ce que** les composants du noyau sont mélangés entre eux, granulés et mis sous forme de comprimés, le noyau ainsi formé est revêtu d'une enveloppe et l'enveloppe est finalement munie d'une ou plusieurs ouvertures.

34. Forme d'administration pharmaceutique selon l'une des revendications 28 et 29, consistant en un système osmotique à deux chambres comprenant un noyau avec une couche de principe actif contenant
• 1 à 40 % de principe actif (I),
• 50 à 95 % d'un ou plusieurs polymères actifs du point de vue osmotique,
et une couche d'osmose contenant
• 40 à 90 % d'un ou plusieurs polymères actifs du point de vue osmotique
• 10 à 40 % d'un additif actif du point de vue osmotique,
ainsi qu'une enveloppe, consistant en un matériau perméable à l'eau, imperméable aux composants du noyau, avec au moins une ouverture.

35. Forme d'administration pharmaceutique selon la revendication 34 qui contient dans le noyau, dans la couche de principe actif, du poly(oxyde d'éthylène) d'une viscosité de 40 à 100 mPa.s (solution aqueuse à 5 %, 25°C) comme polymère actif du point de vue osmotique et qui contient dans le noyau, dans la couche d'osmose, du poly(oxyde d'éthylène) d'une viscosité de 5 000 à 8 000 mPa.s (solution aqueuse à 1 %, 25°C) comme polymère actif du point de vue osmotique.

36. Forme d'administration pharmaceutique selon l'une des revendications 34 et 35, **caractérisée en ce que** l'enveloppe consiste en acétate de cellulose ou en un mélange d'acétate de cellulose et de polyéthylèneglycol.

37. Procédé pour produire un système osmotique à deux chambres, tel que défini dans l'une des revendications 34 à 36,
**caractérisé en ce que**
• les composants de la couche de principe actif sont mélangés et granulés et
• les composants de la couche d'osmose sont mélangés et granulés,
• puis les deux granulats sont compressés en un comprimé à deux couches sur une presse à comprimés à deux couches,
• le noyau ainsi formé est ensuite revêtu de l'enveloppe et
• l'enveloppe est munie d'une ou plusieurs ouvertures du côté du principe actif.

38. Médicament contenant une forme d'administration pharmaceutique applicable par voie orale, solide, avec une libération du principe actif (I) modifiée, définie comme dans la revendication 1.

39. Utilisation d'une forme d'administration pharmaceutique contenant le principe actif (I) applicable par voie orale, solide, à libération modifiée, définie comme dans la revendication 1, pour la production d'un médicament pour la prophylaxie, la prophylaxie secondaire et/ou le traitement de maladies.

40. Utilisation selon la revendication 39 pour la production d'un médicament pour la prophylaxie, la prophylaxie secondaire et/ou le traitement des maladies thromboemboliques.

41. Utilisation selon la revendication 40 pour la production d'un médicament pour la prophylaxie, la prophylaxie secondaire et/ou le traitement de l'infarctus du myocarde, de l'angine de poitrine, des réocclusions et resténoses après une angioplastie ou un pontage aortocoronarien, de l'apoplexie cérébrale, des attaques ischémiques transitoires, des maladies d'occlusion artérielles périphériques, des embolies pulmonaires ou des thromboses veineuses profondes.

42. Utilisation du 5-chloro-*N*-({(5*S*)-2-oxo-3-[4-(3-oxo-4-morpholinyl)-phényl]-1,3-oxazolidin-5-yl}-méthyl)-2-thiophène-carboxamide (I) pour la production d'une forme d'administration pharmaceutique telle que définie dans la revendication 1.
